Europäisches Patentamt

European Patent Office

Office européen des brevets

(19)

(11) Publication number: **0 215 539**
A2

(12) # EUROPEAN PATENT APPLICATION

(21) Application number: **86302467.5**

(22) Date of filing: **03.04.86**

(51) Int. Cl.⁴: **C 12 N 15/00, C 12 N 1/14**

(30) Priority: **04.04.85 GB 8508800**

(43) Date of publication of application: **25.03.87**
**Bulletin 87/13**

(84) Designated Contracting States: **AT BE CH DE FR GB IT LI LU NL SE**

(71) Applicant: **GLAXO GROUP LIMITED, Clarges House 6-12 Clarges Street, London W1Y 8DH (GB)**

(72) Inventor: **Fraser, Madeleine Jane, 111 Upperthong Lane, Holmfirth West Yorkshire (GB)**
Inventor: **Foster, Stephen George, 12, North Street Steeple Bumpstead, Near Haverhill Suffolk (GB)**

(74) Representative: **Hildyard, Edward Martin et al, Frank B. Dehn & Co. Imperial House 15-19 Kingsway, London WC2B 6UZ (GB)**

(54) **Plasmids and components thereof for use in filamentous fungi.**

(57) The present invention provides plasmids suitable as vector systems for filamentous fungi, especially antibiotic-producing filamentous fungi such as antibiotic-producing strains of Acremonium chrysogenum or Penicillium chrysogenum, comprising an autonomous replication sequence which is effective in the intended host, an activator sequence derived from, or copied from, DNA of the intended host and/or one or more other fungi of the same species, and a fungal selectable protein-coding element, transcription of which is directed by the activator sequence.

IL 50 327

## Microbiological process and product

The present invention relates to novel plasmids and to novel components thereof and processes for their production. In particular, it relates to novel plasmids which constitute vector systems suitable for use in filamentous fungi, especially in antibiotic-producing species such as Acremonium chrysogenum and Penicillium chrysogenum.

Strain improvement in antibiotic-producing species of filamentous fungi has previously been dependent mainly on isolation of suitable wild strains, followed by mutation and selection. The incidence of desirable mutations is randomly distributed and extremely low and there is a need therefore for a means whereby specific desirable genetic characteristics can be introduced into filamentous fungi. We have now developed a plasmid vector system for this purpose.

Plasmids capable of acting as efficient vector systems in E. coli are well-known and have been extensively used to modify the genotype of E. coli strains. Vector systems are also well-known for various other commonly transformed organisms, such as Saccharomyces cerevisiae. In contrast, very little is known about vector systems capable of transforming filamentous fungi. To be useful, such vector systems must exhibit an acceptably high transformation frequency and must be so constructed that the desired coding elements introduced into the host organism are efficiently expressed. In view of the lack of knowledge in the art as to how such requirements can be met, we have adopted an essentially empirical approach for construction of plasmids of value as vector systems for filamentous fungi, involving the use of novel isolation techniques for certain components.

It is known that plasmids are capable of efficiently transforming filamentous fungi only if they include an element which enables them to replicate in the host (hereinafter referred to as an autonomous replication sequence, or ARS). Plasmids which lack an ARS can transform host cells only at very low frequency by integration into the genome. For a plasmid to be a suitable vector system for a filamentous fungus, it must include, in addition to an ARS, a fungal selectable protein coding element and a further element which directs expression of this coding element (hereinafter referred to as an activator sequence). In our studies, we have found it convenient to fuse a chosen selectable protein-coding element, generally an E. coli-derived selectable protein-coding element, with a suitable activator sequence to form a composite element, commonly referred to as a selectable marker.

Experiments which we have carried out have shown that the normal promoter regions of chosen E. coli-derived fungal selectable protein-coding elements are not functional in filamentous fungi, such as Acremonium chrysogenum and Penicillium chrysogenum. Surprisingly, we have also found that DNA restriction enzyme fragments, which are suitable as activator sequences for yeast vector systems, are not necessarily capable of functioning as activator sequences when introduced into cells of a filamentous fungus as a suitably-sited component of a plasmid comprising an ARS and fungal selectable protein coding element. In addition, we have found that restriction enzyme fragments from the genome of Penicillium chrysogenum, which can be used as activator sequences in the construction of vector systems for this species, will not necessarily function as an activator sequence when incorporated appropriately into plasmids comprising an ARS and fungal selectable protein-coding elements which are then used to transform Acremonium chrysogenum.

On the basis of the work outlined above, we have found that the nature of the DNA sequences which can be effectively incorporated in a plasmid vector for use in filamentous fungi is highly critical. Specifically, we have found that it is highly desirable, when constructing a vector system for a particular strain of a filamentous fungus to use DNA sequences derived from, or copied from, the genome of the intended host and/or one or more other fungi of the same species for selection of suitable activator sequences. We believe that isolated activator sequences derived from, or copied from, DNA of filamentous fungi are novel and such sequences therefore form one aspect of the present invention. It should be understood that the term 'copied' as used herein refers both to direct copying of DNA using appropriate enzymes and indirect copying of DNA by in vitro chemical synthesis.

According to a further aspect of the present invention, we provide a plasmid suitable as a vector system for a filamentous fungus, especially an antibiotic-producing filamentous fungus such as an antibiotic-producing strain of Acremonium chrysogenum or Penicillium chrysogenum, said plasmid comprising an autonomous replication sequence which is effective in the intended host, an activator sequence derived from, or copied from, DNA of the intended host and/or one or more other fungi of the same species, and a fungal selectable protein-coding element, transcription of which is directed by the activator sequence.

A vector system according to the invention will generally include a viral vector or a portion of a 5-15kb prokaryotic plasmid, conveniently an E. coli-derived plasmid. If a prokaryotic plasmid is used, the portion of the prokaryotic plasmid selected will preferably include those sequences which enable replication and selection in the prokaryote. The ARS sequence for construction of a vector system according to the invention, which enables replication in the intended host and efficient transformation, will most preferably be derived from isolated total DNA of the intended host and/or one or more other fungi of the same species.

One or more protein-coding sequences may be under the control of the fungal activator sequence. The fungal selectable protein coding element will generally be fused with the fungal activator sequence and can be any DNA sequence which codes for a protein which can be readily directly or indirectly detected in fungal cells, e.g. a sequence coding for a protein which confers resistance to an antibiotic, or one which causes the production of a pigment such as the protein coding region of the tyrosinase gene from Streptomyces antibioticus which confers the ability to produce the dark pigment melanin. Conveniently, a vector system according to the invention may, for example, contain the coding region of a neomycin-phosphotransferase gene joined to the fungal activator sequence. Fungal cells transformed with such a vector system may be selected using an antibiotic which is normally effective in untransformed fungal cells and which is inactivated when phosphorylated by neomycin phosphotransferase, for example, the aminoglycoside antibiotic G418. It is feasible, in some cases, for the fungal selectable protein coding element to be a coding element required for a desired improvement of the phenotype of the intended host.

Since the genomes of filamentous fungi have not been well-characterised, we have derived activator sequences and autonomous replication sequences from isolated total DNA of such fungi by methods involving functional selection. These methods also constitute aspects of the present invention.

Our preferred method for isolating a fungal activator sequence comprises the steps of:

i)    digestion of total DNA isolated from the intended host and/or one or more fungi of the same species with one or more restriction enzymes;

ii)   preparation of plasmids known to be capable of replicating in a chosen eukaryote and which include an appropriate selectable marker for the same organism and also a fungal selectable protein coding element fused with a restriction fragment obtained in (i);

iii)  transformation of the chosen eukaryotic cells with the plasmids prepared in (ii) and selection of transformants using the selectable marker;

iv)   selection of transformants obtained in (iii) which exhibit expression of the fungal selectable protein coding element; and

v)    isolation of the plasmid containing the desired activator sequence and optionally cloning that plasmid in E. coli.

The eukaryote chosen for transformation in step (iii) is most desirably, if feasible, the intended host for the vector system under construction or another filamentous fungus selected from the strains suitable as a source of DNA in step (i). If such a fungus has not previously been transformed with a vector system, it is preferable to use a commonly transformed eukaryote, preferably a yeast of the genus Saccharomyces such as Saccharomyces cerevisiae. The DNA fragments produced in step (i) will preferably be 0.5-1 kb. All the plasmids may be prepared using recombinant DNA techniques which per se are well known in the art. The coding sequence for neomycin phosphotransferase may be chosen, for example, as the fungal selectable protein coding element and transformants in step (iv) may be selected for expression of this coding element using, for example, the antibiotic G418. The coding sequence for neomycin phosphotransferase may be conveniently derived from, for example, the transposon Tn5. The selected activator sequence may be incorporated into a vector system according to the invention, with or without a fused fungal selectable protein coding element, using recombinant DNA techniques known per se. All the other constructional elements may also be incorporated using conventional recombinant DNA techniques.

Our preferred method for isolating a fungal ARS comprises the steps of:

i)    digestion of total DNA isolated from the intended host and/or one or more fungi of the same species with one or more restriction enzymes;

ii)   insertion of the fragments into a plasmid which cannot replicate in a chosen eukaryote and which includes a known appropriate selectable marker for the same organism; and

iii)  selection of plasmids which will transform the chosen eukaryote at high frequency and optionally cloning the selected plasmid with the desired ARS in E. coli.

The most desirable conditions in step (i) and the choice of eukaryote for selection of plasmids are determined by the same factors as for isolation of fungal activator sequences by the method hereinbefore described. All the steps can be carried out using techniques known per se.

In the examples quoted, we have found it particularly useful to use a restriction fragment of 1-5Kb which provides the autonomous replicating activity and contains one or more Bgl-II sites remote from the functional sequence. The incorporation of such fragments into the chosen vector introduced a convenient restriction site for the insertion of the selectable protein-coding element and later the activator sequence.

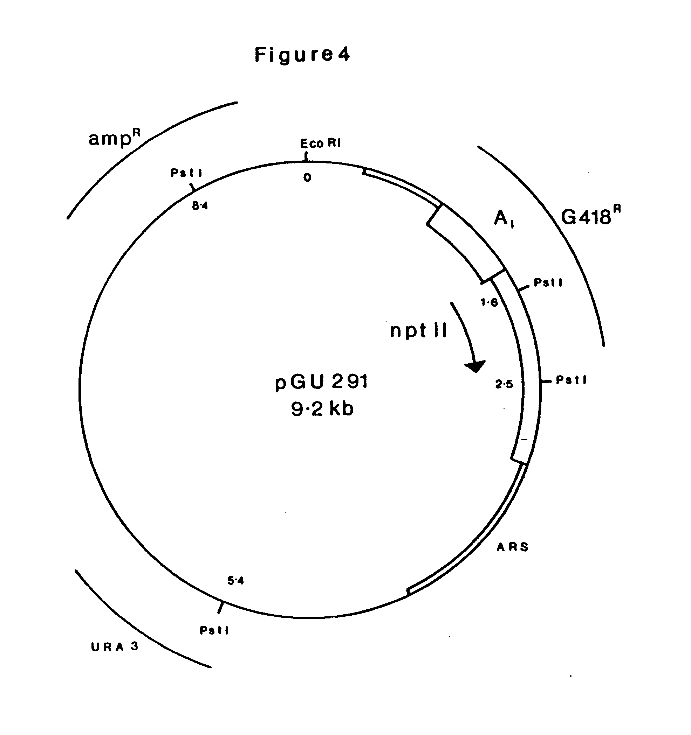

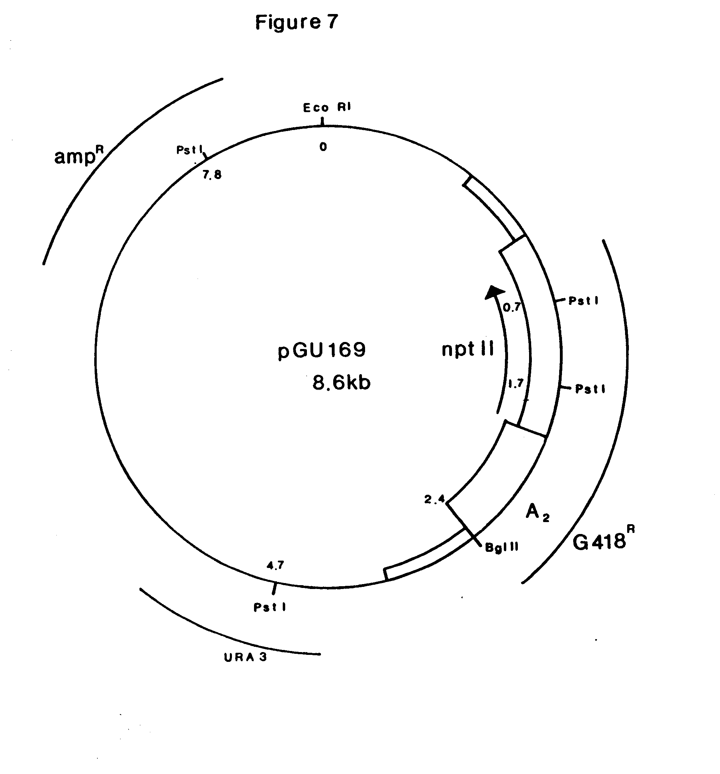

Figures 4 and 7 of the accompanying figures are structural maps of preferred basic vector systems for Acremonium chrysogenum strains and Penicillium chrysogenum strains respectively which possess an ARS and a fungal selectable marker. These vector systems, designated pGU 291 and pGU 169, are useful starting points for the construction of further vector systems with the same ARS and activator sequence but with one or more additional genetic elements, which are suitable for improving a strain of Acremonium chrysogenum or Penicillium chrysogenum with regard to antibiotic-production.

Vector systems according to the invention may be constructed for a number of purposes. Such vector systems may, for example, be used to modify the genotype of antibiotic-producing filamentous fungi, such as antibiotic-producing strains of Acremonium chrysogenum and Penicillium chrysogenum, to increase antibiotic production, to change growth characteristics (e.g. to enable growth on novel or alternative carbon sources) or to cause the biosynthesis of novel useful products.

According to another feature of the present invention, we therefore provide a method of modifying the genotype of filamentous fungi such as antibiotic-producing strains of Acremonium chrysogenum or Penicillium chrysogenum, which comprises transformation with an appropriate vector system according to the invention.

In order to transform fungal cells, the cells may first be converted to protoplasts or, alternatively treated with a metal salt such as lithium acetate for example, as described by Dhawale, S.S., Paiepta, J.V. and Marzluf, G.A. in Current Genetics (1984) 8, pp. 77-79. A suitable method for protoplast generation from Acremonium chrysogenum is given in our British Patent No. 2,021,640 and similar methods, well known to those in the art, are applicable to other filamentous fungi.

The following non-limiting Examples illustrate the present invention.

Materials

Chloramphenicol, TRIS buffer (tris[hydroxymethyl]aminomethane), EDTA (ethylenediamine tetraacetic acid), sodium dodecyl sulphate (SDS), ethidium bromide, agarose (type II), spermidine, 8-hydroxyquinoline, β-mercaptoethanol, dATP, dithiothreitol, RNAase, adenine, β-glucuronidase, polyethylene glycols, tryptophan and ampicillin were supplied Sigma (London) Chemical Company.

All other chemicals were AnalaR grade from BDH Chemicals Ltd.

Tryptone, yeast extract, yeast nitrogen base and bacto-peptone were supplied by Difco Laboratories, Michigan, USA.

Tryptone soya broth, caesin acid hydrolysate, peptone and agar (number 3) were from Oxoid Ltd, London.

T4 DNA ligase, calf intestinal phosphatase (molecular biology grade) were supplied from the Boehringer Corporation (London) Ltd.

Restriction enzymes Bgl II, Sau 3A and Pst I were supplied by P and S Biochemicals Ltd, Liverpool. λ CI 857 Sam 7 digested with Hind III (λ/ Hind III) and Bam HI were supplied by BRL (UK) Ltd, Cambridge.

Novozyme 234 was supplied by Novo Enzyme Products Ltd, Windsor. G418 was supplied by Gibco Ltd, Paisley.

Strains and Vectors

Acremonium chrysogenum and Penicillium chrysogenum were used as sources of chromosomal DNA, and as recipients of hybrid plasmid DNA, suitable strains being ATCC 14553 and ATCC 10002.

E. coli NCIB 12046 was used as the source of pBR322::Tn5 DNA.

E. coli NCIB 11933 was used as a recipient of hybrid plasmid DNA.

Saccharomyces cerevisiae NCYC 1549 was used as the recipient for hybrid plasmid DNA.

The vector used was the pBR322 derivative YIp5, E. coli ATCC 37061.


Results


The results obtained are illustrated by reference to the accompanying drawings, in which:


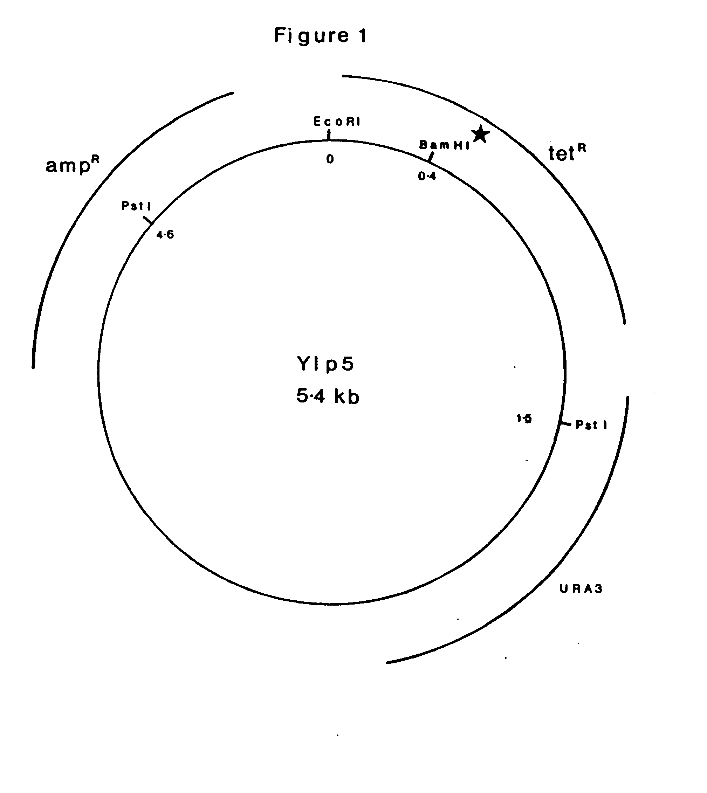

Figure 1 represents the plasmid YIp5. This plasmid confers tetracycline resistance (tet ) and ampicillin resistance (amp ) on E. coli NCIB 11933 and uracil prototrophy (URA-3) at low frequencies on S. cerevisiae NCYC 1549. The star indicates the Bam HI restriction endonuclease site wherein A. chrysogenum and P. chrysogenum Sau 3A - digested DNA were inserted giving rise to pGU270 (figure 2, A. chrysogenum DNA) or pGU253 (figure 5, P. chrysogenum DNA).


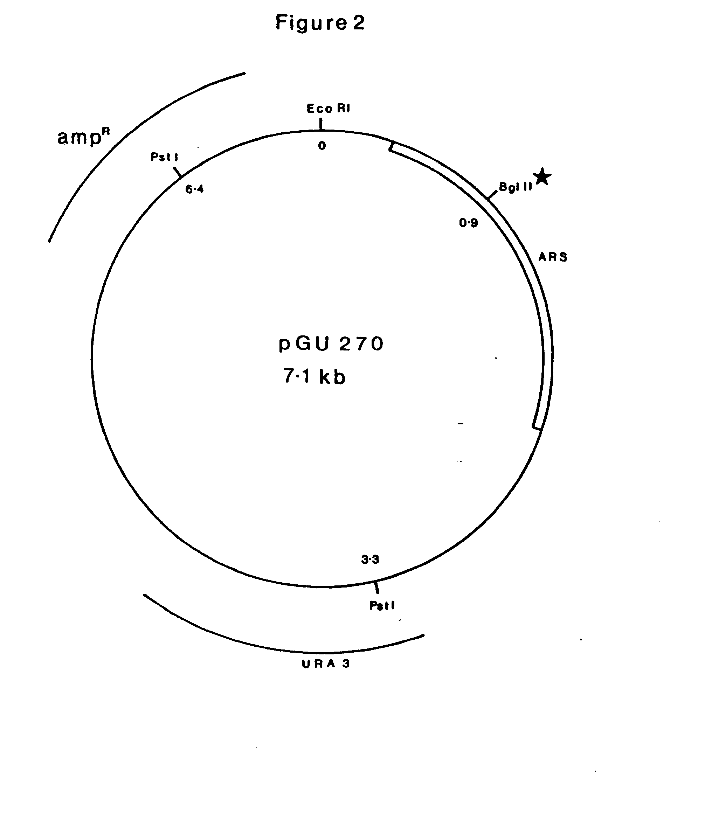

Figure 2 represents the plasmid construct pGU270. This plasmid confers ampicillin resistance (amp$^R$) on E. coli NCIB 11933. Tetracycline resistance has been abolished by insertional inactivation with the addition of a DNA sequence (ARS) which allows the plasmid to transform S. cerevisiae NCYC 1549 to uracil prototrophy (URA-3) at high frequencies. The star indicates the Bgl II restriction endonuclease site wherein the npt II gene from Tn5 on a Bgl II - Bam HI fragment was inserted to give pGU281.


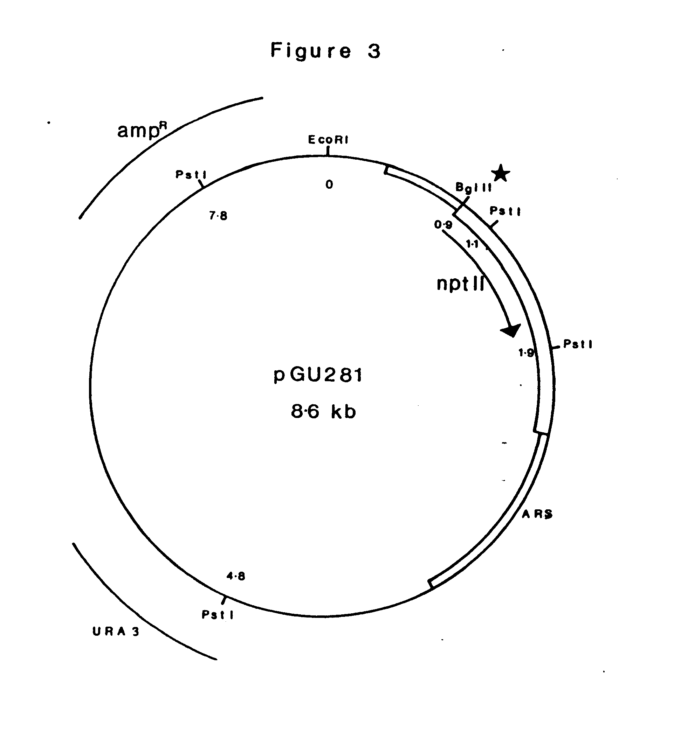

Figure 3 represents the plasmid construct pGU281. This plasmid confers ampicillin resistance (amp ) on E. coli NCIB 11933, and transforms S. cerevisiae NCYC 1549 to uracil prototrophy (URA-3) at high frequencies. The construct includes the structural gene for neomycin phosphotransferase (npt II) from Tn 5. The direction of gene expression is shown by the arrow. The star indicates the Bgl II restriction endonuclease site wherein A. chrysogenum or P. chrysogenum Sau 3A-digested DNA could be inserted to activate the cryptic npt II gene.


Figure 4 represents the plasmid construct pGU291. This plasmid confers ampicillin resistance (amp ) on E. coli NCIB 11933 and transforms S. cerevisiae NCYC 1549 on uracil prototrophy (URA-3) and G418 resistance (G418 ). The construct contains DNA (A$_1$) additional to pGU281 (figure 3) which allows expression of the cryptic npt II gene. The plasmid can also transform A. chrysogenum to G418 resistance.

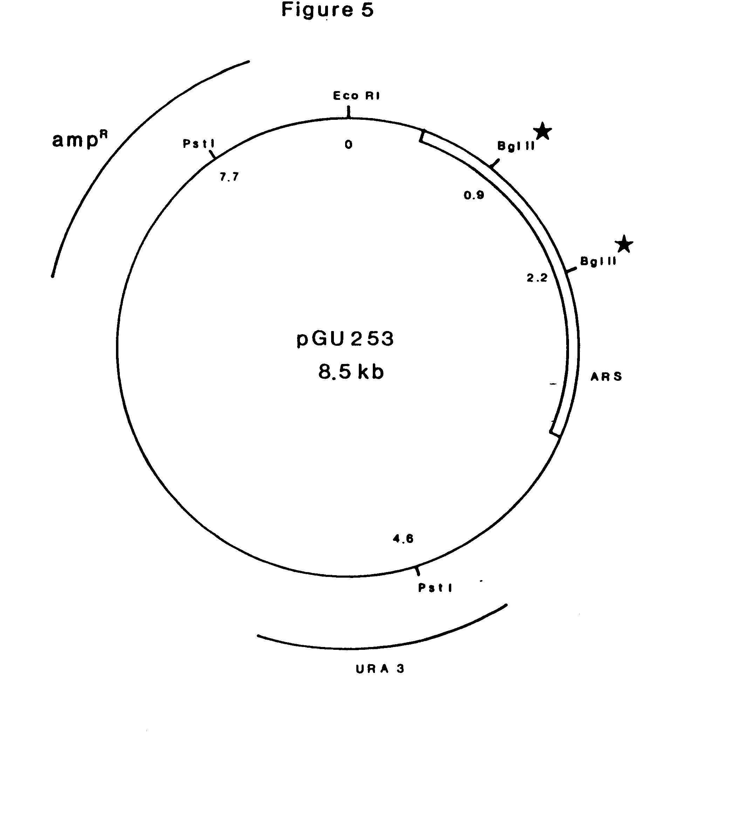

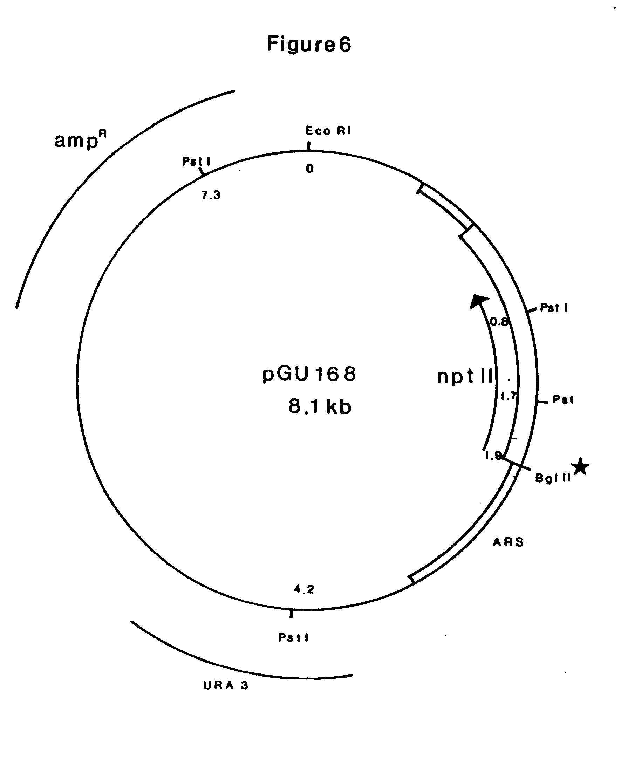

Figure 5 represents the plasmid construct pGU253. This plasmid confers ampicillin resistance (amp ) to E. coli NCIB 11933. Tetracycline resistance has been abolished by the addition of the DNA sequence ARS through insertional inactivation. The ARS DNA insertion allows the plasmid to transform S. cerevisiae NCYC 1549 to uracil prototrophy (URA 3) at high frequencies. The stars indicate the Bgl II restriction enzyme endonuclease sites flanking the fragment replaced by the npt II gene from Tn 5 on a Bgl II - Bam HI fragment to give pGU168 (figure 6).

Figure 6 represents the plasmid construct pGU168. This plasmid confers ampicillin resitance (amp ) to E. coli NCIB 11933 and transforms S. cerevisiae NCYC 1549 to uracil prototrophy (URA-3) at high frequencies. The construct includes the structural gene for neomycin phosphotransferase (npt II) derived from Tn 5. The direction of gene expresssion is shown by the arrow. The star indicates the Bgl II restriction endonuclease site wherein P. chrysogenum Sau 3A-digested DNA could be inserted to activate the npt II gene.

Figure 7 represents the plasmid construct pGU169. This plasmid confers ampicillin resistance (amp ) on E. coli NCIB 11933 and transforms S. cerevisiae NCYC 1549 to uracil prototrophy (URA-3), and G418 resistance (G418 ). The construct contains DNA additional to pGU168 (figure 6) which allows the expression of the cryptic npt II gene (A). This plasmid can also transform P. chrysogenum to G418 resistance.

Example 1

Construction of a Plasmid Capable of Autonomous Replication in S. cerevisiae

A)    Cloning Random DNA Fragments into YIp5

i)    Large Scale Plasmid Preparation of YIp5

E.coli ATCC 37061 harbouring YIp5 was grown in 1 1. of L broth (1% tryptone, 0.5% yeast extract, 0.5% NaCl) with shaking at 37°C. At an optical density $(OD)_{550}$ of 0.6, chloramphenicol was added to 100 ug/ml final concentration. After an additional 17 hours incubation at 37°C, the bacteria were sedimented at 10,000g for 10 minutes. The bacterial pellet was resuspended in 20 mls of 50mM glucose, 25mM TRIS-HCl (pH 8.0), 10mM EDTA, and held at 21°C for 5 minutes. This was followed by 40 mls of 0.2N NaOH, 1% SDS. The contents were mixed by gentle inversion, and held on ice for 10 minutes. 30 mls of ice-cold, 5M potassium acetate was added to the mixture, and the contents mixed by violent inversion, then held on ice for 10 minutes. The lysate was centrifuged at 50,000g for 20 minutes, the resulting supernatant collected and retained. 0.6 volumes of isopropanol was added to the supernatant, and, after mixing, held at 21°C for 15 minutes. DNA was sedimented at 12,000g for 30 minutes. The pellet was washed in 70% ethanol, and finally dried in a vacuum. The pellet was resuspended in 8 mls of 50mM TRIS-HCL (pH 8.0), 1mM EDTA (pH 8.0). To this was added 8g of solid caesium chloride, and ethidium bromide to 0.6 mg/ml. The mixture was dispensed into centrifuge tubes and centrifuged for 36 hours at 140,000g at 15°C. The plasmid band was observed by illumination with long-wave ultra-violet light, and removed with a needle and syringe. Ethidium bromide was removed by extraction with isoamyl alcohol. Caesium chloride was removed by dialysis against 100 volumes of 10mM Tris-HCl, 10mM NaCl 0.1mM EDTA (pH 8.0), (TE) at 4°C, repeated every 4 hours.

Plasmid DNA concentration was determined by measuring the $OD_{260}$ of the solution (An $OD_{260}$ of 1.0 in a path-length of 1 cm is equivalent to 50 ug/ml DNA).

ii) **Digestion of YIp5 with Bam HI**

5 ug of YIp5 was dissolved in 20 ul of reaction buffer, with a final concentration of: 50mM NaCl; 10mM TRIS-HCl (pH 7.5); 10mM $MgCl_2$; 1mM dithiothreitol. Restriction endonuclease was added to 1 unit per ug DNA. Complete digestion was achieved after 2 hours, monitored using gel electrophoresis on 0.8%

agarose slab gels (8 cm x 10 cm x 0.4 cm). A 0.2 ug DNA sample in 0.4M urea; 5% (w/v) sucrose; 5mM EDTA; 0.01% bromophenol blue was loaded onto the gel. Samples were run for 1 hour at 100 mA. The gel was stained in 0.5 ug/ml ethidium bromide for 15 minutes, viewed on a transilluminator (Fotodyne UV300) and photographed using Type 667 Land film on a Polaroid CU-5 camera with a Kodak 22A Wratten filter.

### iii) Dephosphorylation of Digested YIp5

5 ug of Bam HI-digested YIp5 was treated with 5 units of calf intestinal alkaline phosphatase at 37°C for 1 hour in 50mM TRIS HCl (pH 9.0), 1mM $MgCl_2$ , 0.1mM $ZnCl_2$ , 1mM spermidine. After 1 hour the reaction was made up to 100 ul in 10mM TRIS-HCl (pH 8.0), 100mM NaCl, 1mM EDTA, and 0.5% SDS. The mixture was heated to 68°C for 15 minutes, and extracted twice with an equal volume of phenol: chloroform (1 volume of phenol, containing 0.1% 8-hydroxyquinoline and equilibrated with 0.1M TRIS-HCl (pH 8.0),0.2% B-mercaptoethanol: 1 volume of chloroform). The aqueous phase was then extracted twice with ether.

The DNA solution was adjusted to 0.3M sodium acetate, to which two volumes of ethanol were added. The solution was mixed and held at -25°C for 30 minutes. DNA was sedimented at 13,000g for five minutes, the supernatant discarded, and the DNA pellet dried under vacuum.

The DNA was dissolved in 5 ul of TE buffer. Confirmation that all molecules were dephosphorylated was as follows. 100 ng of Bam HI-digested and 100 ng of BamHI-digested, calf intestinal phsophatase treated YIp5 were adjusted to 20mM TRIS-HCL (pH 7.6), 10mM $MgCl_2$ , 10mM dithiothreitol, 0.6mM dATP,, 0.1 unit T4-DNA ligase. The reactions were held at 14°C for 16 hours after which they were incubated at 70°C for 10 minutes, and rapidly cooled to 4°C. The reactions underwent gel electrophoresis as described in A (ii). YIp5 was judged to dephosphorylated by the inability of the treated molecule to reform circular molecules in the presence of T4 DNA ligase.

iv) <u>Large Scale DNA Preparation of Acremonium chrysogenum DNA</u>

<u>A.chrysogenum</u> mycelial fragments were inoculated to 100 mls growth medium (Sucrose, 3.0g; glucose, 15.0g; NaCl, 0.5g; $K_2HPO_4$, 0.5g; $MgSO_4.7H_2O$, 0.5g; $FeSO_4.7H_2O$, 0.01g; Tryptone Soya Broth, 5.0g; Peptone, 1.0g; Yeast Extract, 2.0g, water to 1000 mls) to a final concentration of $2 \times 10^6$ colony forming units per ml. The culture was incubated for 48 hours at 25.5°C and 200 rpm after which it was used to inoculate 900 mls of the same medium. After a further 48 hour incubation in the same conditions, the mycelium was harvested at 3000g for 1 hour. The mycelium was washed twice in 100 mls sterile distilled water, and resuspended in 100 mls 0.5% B-mercaptoethanol. The mycelium was incubated at 25.5°C for 30 minutes, harvested and washed twice in 100 mls 0.7M KCl, sodium phosphate buffer (pH 5.8) (stabilisation buffer). The mycelium was resuspended in 5 mg/ml Novozyme 234 in a final volume of 10 mls stabilisation buffer/2g of mycelium. The culture was incubated at 25.5°C for 2 hours at 220 rpm to liberate protoplasts. After protoplasting the culture was washed twice in stabilisation buffer, and resuspended in 5 mls of 100mM EDTA, 1% SDS, 100 mg/ml proteinase K. The mixture was held at 37°C for 2 hours, then moved to 65°C for 10 minutes and allowed to equilibrate to room temperature. Phenol, 0.1% 8-hydroxyquinoline equilibrated with 0.1M TRIS-HCl (pH 8.0) and 0.2% B-mercaptoethanol was added to an equal volume, and the phases mixed by gentle inversion. This was followed by the addition of 5 mls chloroform, mixing again achieved by gentle inversion. Partition of the phases was achieved by centrifugation at 3,000g for 5 minutes, and the non-aqueous phase discarded. The aqueous phase was re-extracted with phenol and chloroform as described. The aqueous phase was then extracted twice with an equal volume of chloroform.

Preheated RNAse was added to the aqueous phase at 40 ug/ml, and incubated for 1 hour at 37°C. The solution was adjusted to 0.3M sodium acetate, and the DNA precipitated with 2 volumes of ethanol for 1 hour at -25°C. DNA was sedimented at 10,000g for 30 minutes, the supernatant discarded, and the pellet washed in 70% ethanol. The pellet was dried under

- 15 -

0215539

vacuum, and resuspended in TE buffer (pH 8.0).

The DNA concentration was determined as described in A i).

v) <u>Determination of Ideal Conditions for Partial Digestion of A. chrysogenum DNA with Restriction Endonuclease Sau 3A</u>

1 ug of chromosomal DNA was digested with: 1, 0.5, 0.25, 0.125, 0.0625, 0.035, 0.002 units of <u>Sau</u> 3A for one hour at 37°C in 50mM NaCl, 10mM TRIS-HCl (pH 7.5). 10mM $MgCl_2$, 1mM dithiothreitol. DNA digestion was observed using gel electrophoresis described in A(ii). Fragment size distribution was assessed with $\lambda$ <u>Hind</u> III. Conditions suitable to give chromosomal fragments of 5 kb were decided (0.09 units/hr/ug).

vi) <u>Large Scale Digestion of Chromosomal DNA</u>

100 ug of <u>A.chrysogenum</u> DNA were treated by the conditions described in A v). After 1 hour at 37°C the reaction was stopped by heating to 70°C for 10 minutes followed by rapid cooling to 4°C. The digestion of chromosomal DNA was confirmed by gel electrophoresis described in A ii). The sample was phenol extracted, ether extracted and ethanol precipitated as described in A ii).

vii) <u>Ligation of YIp5 and A. chrysogenum DNA</u>

5 ug of cut YIp5 from A iii) and 16 ug of chromosomal DNA from A vi) were added to 400 ml of ligation buffer containing 20mM TRIS-HCl (pH 7.6), 10mM $MgCl_2$, 10mM dithiothreitol, 0.6mM ATP, 5 units T4 DNA-ligase and incubated for 72 hours at 14°C.

viii) <u>Transformation of S. cerevisiae to Uracil Prototrophy</u>

The DNA mixture derived from steps A i)-viii) was used to transform <u>S.cerevisiae</u> NCYC 1549.

A culture of <u>S.cerevisiae</u> NCYC 1549 was grown overnight at 30°C in 50 mls of 1% yeast extract 2% Difco Bacto Peptone, 2%

glucose and 20 mg/l adenine.  Cells were harvested at 6,000g for 10 minutes, and washed twice with sterile distilled water. The cells were resuspended in half the original volume of 1M sorbitol, 25mM EDTA (pH 8.0), 50mM dithiothrieitol.  The mixture was incubated at 30°C for 30 minutes with gentle agitation. Cells were subsequently sedimented at 6,000g for 10 minutes, then washed twice with 1.2M sorbitol.

The fungal pellet was resuspended in half the original culture volume of 1.2M sorbitol, 0.01M EDTA, 0.1M sodium citrate (pH 5.8), 1% (v/v) B-glucuronidase.  The culture was incubated for 2 hours at 30°C to liberate sphaeroplasts.  Sphaeroplasts were sedimented at 4,000g for 10 minutes, and gently resuspended in 1.2M sorbitol.  This step was repeated 3 times. The sphaeroplasts were finally resuspended in 1.2M sorbitol, 10mM $CaCl_2$ to give $1 \times 10^{10}$ sphaeroplasts/ml.

2 ug of the ligation mixture from A i)-viii) was added to $5 \times 10^8$ sphaeroplasts.  After gentle mixing, these were left for fifteen minutes at 21°C.  Added to the mixture then was 0.5 mls 20% polyethylene glycol 4000, 10mM CaCl , 10mM TRIS-HCl (pH 7.5).  After 5 minutes the sphaeroplasts were sedimented at 6000g for 10 seconds, the supernatant discarded, and the sphaeroplasts resuspended in 1 ml of 1.2M sorbitol.

The suspension was divided into 0.1 ml aliquots and added to 7 mls of agar; 3% oxoid no.3 agar, 0.67% Difco Yeast Nitrogen Base, 2% glucose, tryptophan 20 ug/l stabilised to 1.2M sorbitol (pH 6.5).  This agar was plated onto a 2% agar of the same composition.  The plates were incubated for 4 days at 30°C.

The selection procedure was for uracil prototrophs.  The plasmid YIp5 contains the URA-3 gene from S.cerevisiae, YIp5 does not have an origin of replication (Autonomous Replication Sequence, ARS)  the aim of this experiment was to complement the missing ARS function with the DNA cloned from A.chrysogenum.

Several URA-3 transformants were observed which were analysed as described below.


ix) Recovery of Transforming DNA from S. cerevisiae

S.cerevisiae NCYC 1549 URA-3 transformants were inoculated to 10 mls of 0.67% Difco yeast nitrogen base, 2% glucose, 20 ug/ml tryptophan, and incubated at 27.5°C for 48 hours. The cells were sedimented at 6000g for 10 minutes, and resuspended in 1.0 ml of 1.0M sorbitol. The cells were sedimented at 13,000g for 10 seconds, and resuspended in 0.5 mls 1M sorbitol, 50mM potassium phosphate buffer (pH 7.5) 14mM B-mercaptoethanol and 25 units B-glucuronidase. After incubation at 30°C for 30 minutes the sphaeroplasts were sedimented at 3000g for 5 minutes, and resuspended in 0.5 mls of 50mM EDTA (pH 8.5) 0.2% SDS. The mixture was heated to 70°C for 15 minutes, then 50 ml of 5M potassium acetate added. The lysate was held on ice for 1 hour. The precipitate was sedimented at 13,000g for 10 minutes. DNA was precipitated from the supernatant with 2 volumes of ethanol at -25°C for 1 hour, and sedimented at 13,000g for 10 minutes. The supernatant was discarded, and, after drying in a vacuum, was resuspended in 100 ul of 0.2M $CaCl_2$, 0.15M $NaCl_2$, 0.015M Sodium citrate (pH 7.0) ready for E.coli transformation.


x) Bacterial Transformation

2 mls of an overnight culture of E.coli NCIB 11933 grown in 0.02M potassium phosphate buffer (pH 7.0), 0.15M ammonium sulphate, 10mM $MgSO_4 \cdot 7H_2O$, 2nM $FeSO_4$, 0.1% glucose, 0.08% caesin acid hydrolysate, 1 ug/ml aneurin, 20 ug/ml proline was transferred to 23 mls of the same medium. At an $OD_{550}$ of 0.4 - 0.5, the culture was cooled on ice for 10 minutes. Bacteria were sedimented at 6000g for 10 minutes and the pellet washed with 12.5 mls of ice cold 0.1M $CaCl_2$. The Bacteria were finally resuspended in 12.5 mls of ice cold 0.1M $CaCl_2$ and held on ice for 20 minutes. The cells were then pelleted, and resuspended in 2.5 mls ice cold 0.1M $CaCl_2$.


To 0.2 ml of the cell suspension was added 0.1 ml of the DNA mixture described in A (ix). The mixture was held at 42°C for 2 minutes, transferred to ice for 90 minutes after which 1 ml of L broth was added. The cell suspension was held at 21°C for 1 hour, and plated onto L-agar (L-broth containing 1.5% w/v agar) containing ampicillin at 100 µg/ml.

The selection was for transformation to ampicillin resistance, an indication of recovery of YIp5-based DNA from the S.cerevisiae NYCY 1549 transformants described in A iix) - x).

xi) Analysis of Transformants

Plasmid DNA was prepared from the ampicillin resistant transformants thus. A 1 ml culture in L-broth (100 mg/ml ampicillin) was sedimented at 13,000g for 10 seconds and resuspended in 0.1 ml 50mM glucose, 25mM TRIS-HCl (pH 8.0), 10mM EDTA, and incubated at room temperature for 5 minutes. 0.2 mls of 0.2N NaOH, 1% SDS was added, and the lysate held at 4°C for 5 minutes. 0.15 mls of 5M potassium acetate (pH 4.8) was added, and the mixture held at 4°C for five minutes. The precipitate was sedimented at 13,000g for 10 minutes. The supernatant was transferred to a fresh tube, mixed with 1 ml of ethanol, and held at room temperature for 2 minutes. Plasmid DNA was sedimented at 13,000g for 10 minutes. The pellet was washed in 70% ethanol and vacuum dried. The DNA was redissolved in 20 ml of TE (pH 8.0) buffer.

This DNA was used to transform S.cerevisiae NCYC 1549 as described in A viii). Isolated DNA that was capable of transforming S.cerevisiae NCYC 1549 to URA-3 at a high frequency ($10^3$ - $10^5$ transformants per ug of DNA) represented an YIp5:: ars plasmid. One such plasmid was pGU270 (figure 2).

B  Cloning the npt II Gene into pGU270

i) Large Scale Plasmid Preparation of pGU270

The method used was as described in A i) except that E.coli NCIB 11933/pGU270 was substituted for E.coli ATCC 37061.

ii) <u>Digestion of pGU270 with Bgl II</u>

The method used was as described in A ii) except that pGU270 was substituted for YIp5, and in the reaction buffer the NaCl concentration was 10mM, rather than 50mM.

iii) <u>Dephosphorylation of Digested pGU270</u>

The method used was as described in A iii) except that <u>Bgl</u> II digested pGU270 was substituted for <u>Bam</u> HI digested YIp5.

iv) <u>Large Scale Plasmid Preparation of pBR322::Tn5</u>

The methods used was as described in A i) except that <u>E.coli</u>/NCIB 12046 was substituted for <u>E.coli</u> ATCC 37061.

v) <u>Digestion of pBR322::Tn5 with Bgl and Bam HI</u>

The method used was as described in B ii) except that pBR322::Tn5 was substituted for pGU270. Once complete <u>Bgl</u> II digestion was confirmed as described in A ii), the NaCl concentration was increased to 50mM, and <u>Bam</u> HI added to 1 unit/µg DNA. Complete <u>Bam</u> HI digestion was confirmed as described in A ii).

vi) <u>Isolation of the Restriction Fragment Covering the npt II Gene</u>

5 µg of <u>Bam</u> HI, <u>Bgl</u> II digested pBR322::Tn5 was loaded onto a electrophoresis gel and run as described in A ii). After staining with ethidium bromide the gel was examined under U.V. light, and the fragment bearing the <u>npt</u> II coding sequence was excised from the gel.

The gel slice was placed in an Eppendorf tube, frozen in a dry ice-ethanol bath and warmed to room temperature. This step was repeated. Agarose was sedimented by centrifugation at 13,000g for 15 minutes. The supernatant was collected, phenol extracted, ether extracted and ethanol precipitated as described in A iii). After vacuum drying, the pellet was resuspended in 5 µl of TE (pH 8.0) buffer.

vii) <u>Ligation of pGU270 with the npt II fragment</u>

0.5 µg of pGU270 from B iii) was mixed with 1 µg of the <u>npt</u> II fragment produced in B vii) and ligated in conditions described in A vii).

viii) <u>Bacterial Transformation</u>

The material from B vii) was used to transform <u>E.coli</u> NCIB 11933 to ampicillin resistance. The process was as described in A x). The selection was on L-agar, ampicillin at 100 µg/ml.

ix) <u>Analysis of Chimeric Plasmids</u>

Transformants were treated as described in A xi). Plasmid pGU281 was produced by processes Bi) - Bx) (Figure 3).

x) <u>Large Scale Plasmid Preparation of pGU281</u>

The method used was as described in A i) except that <u>E.coli</u>/pGU281 was substituted for <u>E.coli</u> ATCC 37061.

xi) <u>Transformation of S. cerevisiae</u>

2 µg of pGU281 was used to transform <u>S.cerevisiae</u> NCYC 1549 to <u>URA</u>-3 by the method described in A viii).

URA-3 transformants were tested on the selective medium described in A viii) with additional G418 at 250 µg/ml. No <u>URA</u>-3 transformant was resistant to G418 at this level, suggesting that the <u>npt</u> II gene was inactive.

.C <u>Activating the npt gene of pGU281 by the addition of</u>
<u>A. chrysogenum DNA fragments</u>

i) <u>Digestion of pGU281 with Bgl II</u>

5 µg of pGU281 was digested to completion as described in B ii).

ii) Dephosphorylation of pGU281

The DNA vector was dephosphoylated as described in A iii).

iii) Digestion of A. chrysogenum DNA with Sau 3A

10 μg of A.chrysogenum DNA was digested with Sau 3A (10 units) in the buffer conditions described in A v). The reaction was monitored by gel-electrophoresis described in A ii). On completion of the reaction, the DNA was heated to 70°C for 10 minutes and treated henceforth as described in A vi).

iv) Ligation of pGU281 to A. chrysogenum DNA

5 μg of cut pGU281 from C ii) and 16 ug of chromosomal DNA from C iii) were ligated as described in A vii).

v) Transformation of S. cerevisiae

The DNA mixtures derived from steps C i) to C iv) were used to transform S.cerevisiae NCYC 1549 to URA-3 by the method described in A viii).

The colonies transformed to uracil prototrophy were pooled and plated onto G418 resistance selective media described in B xi). Colonies resistant to G418 were collected.

vi) Recovery of Transforming DNA from S. cerevisiae

S.cerevisiae NCYC 1549 URA-3, G418 transformants were treated as described in A ix) to recover transforming DNA.

vii) Bacterial Transformation

Material from C vii) was used to transform E.coli NCIB 11933 to ampicillin resistance as described in A x).

viii) Analysis of Hybrid Clones

Plasmid DNA was prepared from the ampicillin-resistant transformants described in C vii) by the techniques described in A xi).

Plasmid DNA thus produced was used to re-transform S.cerevisiae NCYC 1549 as described in A viii). Selection was for URA-3 followed by G418 resistance confirming cotransformation of these markers. One such plasmid was termed pGU291 (figure 4).

## Example 2

## Transformation of A.chrysogenum to G418 Resistance with pGU291

A Large Scale Plasmid Preparation of pGU291

The process was as described in Example 1 A i) with E.coli NCIB 11933 substituted for E.coli ATCC 37061.

. B Fungal Transformation

i) Transformation of A. chrysogenum to G418 Resistance

A 10 ml slope culture of A.chrysogenum was inoculated to 40 mls of 25 g/1 corn steep liquor; 20 g/1, sucrose; 4.5 g/1, ammonium acetate; 2 g/1, methionine. The culture was shaken for 48 hours at 25.5°C. The mycelium was harvested by vacuum filtration, and washed twice with sterile distilled water. The mycelium were resuspended in 0.7M KCl, 2M sodium phosphate buffer, (pH 5.8), 10mM dithiothreitol. The culture was incubated at 25.5°C for 1 hour. The treated mycelium was harvested by vacuum filtration, washed twice with 0.7M KCl, 0.2M sodium phosphate buffer pH 5.8, and resuspended in 5 mg/ml Novozyme 234. The mixture was incubated at 25.5°C for 2 hours. Protoplasts were harvested through glass sinters (porosity 2). Protoplasts were sedimented by centrifugation at 700g, for 10 minutes and washed twice in 0.7M KCl.

To $5 \times 10^7$ protoplasts was added 5 µg pGU291 DNA in 5mM $CaCl_2$, 5mM KCl. After mixing, PEG 8000 was added to a final concentration of 3%, glycerine to 11mM, $CaCl_2$ to 11mM. The

protoplasts were held on ice for thirty minutes followed by the addition of 2 mls of a 25% PEG 8000 solution (50mM $CaCl_2$, 50mM glycine, pH 7.5). The mixture was held at 20°C for 5 minutes.

5 mls of 0.7M KCl, 0.05M $CaCl_2$ was added to the mixture and held at 20°C for 5 minutes. The protoplasts were then sedimented at 700g for 15 minutes and washed in 0.7M KCl. The protoplasts were resuspended in a minimal volume of 0.7 M KCl, and plated on glycerol 20 g/l; $NaNO_3$ , 2 g/l; NaCl, 2 g/l; $KH_2 PO_4$ , 1 g/l; $MgSO_4 .7H_2 O$, 0.5 g/l; $FeSO_4$ 0.001 g/l; KCl 0.5 g/l solidified with 2% oxoid number 3 agar, stabilised with KCl at 700mM overlayed with a cellulose disc. After 72 hours of incubation at 25.5°C, the discs were transferred to the same medium excluding 0.7M KCl with G418 added to 150 µg/ml. The plates were incubated at 25.5°C for 35 days. After such times G418 resistant transformants were observed.

ii) <u>Recovery of Transforming DNA from A. chrysogenum</u>

Transformants were grown in the medium described in Example 2, B i), with G418 at 50 µg/ml, protoplasts were prepared as described.

Total DNA was prepared as described in example 1 A iv). Suitable dilutions of the DNA were used to transform <u>E.coli</u> NCIB 11933 as described in Example 1 A x). Alternatively, the chromosomal DNA described was partially digested with <u>Bgl</u> II (1 unit/ug in 10mM NaCl, 10mM TRIS-HCl (pH 7.5); 10mM $MgCl_2$, 1mM dithiothreitol, for 10 minutes). After which it was phenol and ether extracted, and ethanol precipitated as described in Example 1 A iii). The DNA pellet was resuspended at 5 µg/ml in 20mM TRIS-HCl (pH 7.6) 10mM $MgCl_2$ , 10mM dithiothreitol, 0.6mM ATP, 1 unit/µg DNA T4-DNA ligase, and incubated for 76 hours.

DNA treated thus was used to transform <u>E.coli</u> NCIB 11933 to ampicillin resistance, as described in example 1 section A x). Transformants were analysed as described in example 1 A xi), and found to contain pGU291.

## Construction of a Plasmid Capable of Autonomous Replication in S.cerevisiae

**A.    Cloning Random DNA Fragments into YIp5**

**i)    Preparation of YIp5**

Plasmid YIp5 was treated as described in Example 1 Ai) - iii).

**ii)    Large Scale Preparation of P.chrysogenum DNA**

Spores of P.chrysogenum were inoculated to 25 mls of growth medium (0.05%, $MgSO_4 \cdot H_2O$; 0.4%, $(NH_4)_2SO_4$; 0.2%, ammonium lactate; 0.2%, Urea; 1.4%, $KH_2PO_4$; 0.5% $K_2SO_4$; 0.01% EDTA; 0.04% $FeSO_4$; 0.04%, $MnSO_4$; 0.04% $ZnSO_4$; 0.02% $CuSO_4$; adjusted to pH 6.5 with KOH, glycerol was added to 2% and yeast extract to 0.4%) to a final concentration of $5 \times 10^6$ spores/ml. The culture was grown at 25.5°C for 24 hours then used to inoculate 500 mls of the same medium. After a further two days growth, mycelium was sedimented at 3,000g for 1 hour, washed twice with 100 mls of sterile water and resuspended in 5 mg/ml Novozyme 234 in a final volume of 10 mls stabilisation buffer/2 g mycelium. Protoplast harvesting, lysis and DNA purification were, from then on, as described in Example 1 A iv).

**iii)    Cloning ARS sequences from P.chrysogenum DNA into YIp5**

All steps in this protocol were equivalent to Example 1 A v) - xi). One plasmid with ARS activity pGU253 (Figure 5) was used for further manipulation.

**B.    Cloning the npt II Gene into pGU253**

**i)    All steps were as described in Example 1, B i) - xii) except that pGU253 was used instead of pGU270. One construct pGU168 (Figure 6) was chosen for further manipulation.**

C.   Activating the npt II Gene of pGU168 by the Addition of P.chrysogenum DNA Fragments

i)   All steps were as described in Example 1, C i) - viii) except that pGU168 was substituted for pGU281, and P.chrysogenum DNA prepared in Example 3 A ii) for A.chrysogenum DNA.   One plasmid isolated, pGU169, was used as described in Example 4.

Example 4

Transformation of P.chrysogenum to G418 resistance with pGU169 DNA

A.   Large Scale Plasmid of PGU169

The process was as described in Example 1 A i) with E.coli NCIB 11933/pGU169 substituted for E.coli ATCC 14553.

B.   Fungal Transformation

i)   Transformation of P.chrysogenum to G418 Resistance

Protoplasts were prepared as described in Example 3, A ii). The protoplasts were then harvested as described in Example 2 B i).

Protoplasts were treated with DNA as described in Example 2 B i).

Protoplasts were finally resuspended in 10 mls of the growth medium described in Example 3 A ii).   After 24 hours growth to allow for protoplast-regeneration, regenerates were sedimented at 1000g for ten minutes resuspended in 1 ml of stabilisation buffer and plated as 0.1 ml aliquots on solidified (with Oxoid number 3 agar at 1.5%) growth medium described in Example 3 A ii).   The selection was for G418 at 300 µg/ml.   After 14 days incubation at 25.5°C G418 resistant transformants were observed.

ii) <u>Recovery of Transforming DNA from P. chrysogenum</u>

Transformants were grown in the medium described in Example 3 A ii) with G418 at 100 µg/ml, DNA was prepared as described in 4A.

Total DNA was treated as described in Example 2 B ii). <u>E.coli</u> NCIB 11933 transformed to ampicillin resistance with such DNA was found to harbour pGU169.

Claims

1.      A plasmid suitable as a vector system for
a filamentous fungus comprising an autonomous replication sequence which is effective in the intended
host, an activator sequence derived from, or copied
from, DNA of the intended host and/or one or more
other fungi of the same species, and a fungal
selectable protein-coding element, transcription
of which is directed by the activator sequence.

2.      A plasmid as claimed in claim 1 suitable
as a vector system for an antibiotic-producing
strain of Acremonium chrysogenum.

3.      A plasmid as claimed in claim 1 suitable
as a vector system for an antibiotic-producing
strain of Penicillium chrysogenum.

4.      A plasmid as claimed in any one of claims
1 to 3 wherein the autonomous replication sequence
is derived from isolated total DNA of the intended
host and/or one or more other fungi of the same
species.

5.      A method of constructing a plasmid as claimed
in claim 4 wherein the autonomous replication sequence
is derived from isolated total DNA of the intended
host and/or one or more other fungi of the same
species by a method comprising the steps of:

i)      digestion of total DNA isolated from the
        intended host and/or one or more fungi of
        the same species with one or more restriction
        enzymes;
ii)     insertion of the fragments into a plasmid
        which cannot replicate in a chosen eukaryote and which includes a known appropriate selectable marker for the same organism; and

iii) selection of plasmids which will transform the chosen eukaryote at high frequency and optionally cloning the selected plasmid with the desired ARS in E. coli.

6. A method of constructing a plasmid as claimed in any one of claims 1 to 4 wherein a restriction fragment of 1-5Kb which provides autonomous replicating activity and contains one or more Bgl-II sites remote from the functional sequence is employed.

7. An isolated activator sequence derived from, or copied from, DNA of a filamentous fungus.

8. An isolated activator sequence as claimed in claim 7 derived from, or copied from, DNA of one or more strains of Acremonium chrysogenum or Penicillium chrysogenum.

9. A method for isolating an activator sequence as claimed in claim 7 which comprises the steps of:

i) digestion of total DNA isolated from a filamentous fungus with one or more restriction enzymes;

ii) preparation of plasmids known to be capable of replicating in a chosen eukaryote and which include an appropriate selectable marker for the same organism and also a fungal selectable protein coding element fused with a restriction fragment obtained in (i);

iii) transformation of the chosen eukaryotic cells with the plasmids prepared in (ii) and selection of transformants using the selectable marker;

iv)    selection of transformants obtained in (iii) which exhibit expression of the fungal selectable protein coding element; and

v)    isolation of the plasmid containing the desired activator sequence and optionally cloning that plasmid in E. coli.

10.    Use of an activator sequence as claimed in claim 7 or claim 8 for construction of a plasmid as claimed in claim 1.

11.    A method of modifying the genotype of a filamentous fungus which comprises transformation with a plasmid as claimed in claim 1.

12.    A filamentous fungus transformed by a plasmid as claimed in claim 1.

13.    An antibiotic produced by culturing an antibiotic-producing strain of a fungus as defined in claim 12.

Figure 1

# Figure 2

amp^R

Eco RI

0

Pst I

6·4

Bgl II ★

0·9

ARS

pGU 270

7·1 kb

3·3

Pst I

URA 3

Figure 3

# Figure 4

# Figure 5

Figure 6

Figure 7